# NEUE EUROPÄISCHE PATENTSCHRIFT

(11) **EP 1 628 606 B2**
(45) Veröffentlichungstag und Bekanntmachung des Hinweises auf die Entscheidung über den Einspruch: **01.01.2020**
(45) Hinweis auf die Patenterteilung: 13.07.2011
(21) Anmeldenummer: 04735284.4
(22) Anmeldetag: 28.05.2004
(51) Int. Cl.: A61F 9/008, A61F 9/01, B23K 26/06, B23K 26/00, A61F 9/009

(54) **VORRICHTUNG ZUM PRÄZISEN BEARBEITEN VON MATERIAL**
HIGH-PRECISION MATERIAL PROCESSING DEVICE
DISPOSITIF DE TRAITEMENT PRECIS D'UN MATERIAU

(30) Priorität: 02.06.2003 US 475583 P; 23.07.2003 US 625157
(43) Veröffentlichungstag der Anmeldung: 01.03.2006
(60) Teilanmeldung: 10010955.2
(73) Patentinhaber: Carl Zeiss Meditec AG, 07745 Jena (DE)
(72) Erfinder: BENDETT, Mark, Ann Arbor, MI 48104 (US); BISCHOFF, Mark Dr., 07749 Jena (DE); GERLACH, Mario, 16540 Hohen Neuendorf (DE); MUEHLHOFF, Dirk, 07751 Kunitz (DE)
(74) Vertreter: Patentanwälte Geyer, Fehners & Partner mbB
(86) Internationale Anmeldenummer: PCT/IB2004/001772
(87) Internationale Veröffentlichungsnummer: WO 2004/105661

(56) Entgegenhaltungen:
- WO-A-01/13838
- WO-A-01/54853
- US-A- 5 656 186
- US-A- 5 993 438
- US-A1- 2002 023 903
- US-B2- 6 555 781
- H. Lubatschowski, A. Heisterkamp, F. Will, A.I. Singh, J. Serbin, A. Ostendorf, O. Kermani, R. Heermann, H. Welling, W. Ertmer: "Medical applications for ultrafast laser pulses", RIKEN REVIEW, INSTITUTE OF PHYSICAL AND CHEMICAL RESEARCH, WAKO,, JP, 1 January 2003 (2003-01-01), pages 113-118, XP009179614, JP ISSN: 0919-3405, DOI: 10.1117/12.486505
- Alexander Heisterkamp: "Einsatz ultrakurzer Laserpulse in der refraktiven Laserchirurgie", , pages 1-156, XP055361433, Retrieved from the Internet: URL:http://d-nb.info/966097009/34 [retrieved on 2017-04-04]
- GALVANAUSKAS A, ET AL.: "GENERATION OF HIGH-ENERGY FEMTOSECOND PULSES IN MULTIMODE-CORE YB-FIBER CHIRPED-PULSE AMPLIFICATION SYSTEMS", Optics Letters, Optical Society of America, US, vol. 26, no. 12, 15 June 2001 (2001-06-15) , pages 935-937, XP001103490, US ISSN: 0146-9592
- ENDERT, H. ET AL: "Novel ultrashort pulse fiber lasers for micromachining applications", , no. 43, January 2002 (2002-01), pages 23-27,
- IMRA Press Release: "Laser Industry 'Grand Slam' Winner: Newcomer IMRA America Received 2 Prestigious Awards for New Ultrafast Fiber Lasers", , 21 February 2001 (2001-02-21),
- "New Products", , 8 January 2001 (2001-01-08), Retrieved from the Internet: URL:www.laserfocusworld.com
- "Kapitel 3.5 Photodisruption" In: NIEMZ, M.H.: "Laser-Tissue Interactions", 1996, Springer Verlag
- LEONG, K.: "Technology report Femtosecond lasers for manufacturing", Industrial Laser Solutions, 2002, pages 11-15,

## Beschreibung

Die Erfindung betrifft eine Vorrichtung zur präzisen Bearbeitung von Material und Gewebe, insbesondere ein Lasergerät zur präzisen, mikrometergenauen Bearbeitung von organischem Material, nämlich der menschlichen Augenhornhaut.

In einem wertvollen Beitrag zum Stand der Technik wird in der Patentschrift DE 197 46 483 der Anmelderin beschrieben, wie mit Mikrometerpräzision bei der großflächigen Bearbeitung von Materialien mit Lasern mit großem Spotdurchmesser (mm - cm) makroskopische Materialmengen ablatiert, verdampft oder geschmolzen werden (CO₂-Laser, Nd:YAG, Excimer...).

In einem weiteren wertvollen Beitrag zum Stand der Technik wird in der Patentschrift DE 197 27 573 der Anmelderin ein Algorithmus beschrieben, wie ein Laserstrahl abgelenkt werden kann, um eine bestmögliche und präzise Bearbeitung von Material zu gewährleisten.

In der US 5, 656, 186 wird ein Verfahren zum Bearbeiten vom Material bei gleichzeitiger Vermeidung oder Minimierung von schädigenden Nebenwirkungen (Schmelzränder, thermische Schädigung, akustische Schockwellen, Rissbildung) durch Wahl einer speziellen Pulsdauer in Abhängigkeit vom Material beschrieben.

Die materialbearbeitende Wirkung des Lasers ist dabei auf den kleinen Raumbereich des Laserfokus (typischerweise einige µm³) beschränkt, in dem die Lichtintensität hoch genug ist, um die Schwelle des optischen Durchbruchs zu überschreiten. Lokalisiert auf dieses Fokusvolumen wird der Zusammenhalt des Materials zerstört und es entsteht eine Kavitationsblase. Wird der Laserfokus für jeden Laserpuls an eine neue Position gelenkt, können lineare, flächige oder dreidimensionale Schnittmuster generiert werden. Der Abstand benachbarter Kavitationsblasen muss am Ende der Bearbeitung etwa ihrem Durchmesser entsprechen, damit das Material entlang der Schnitte leicht mechanisch ablösbar ist.

Die bestehenden Lasergeräte für die Materialbearbeitung mit Femtosekunden-Laserpulsen verwenden regenerative Verstärker mit Repetitionsraten bis 15 kHz, mit denen einzelne Pulse eines Femtosekundenoszillators verstärkt werden. Während der Oszillator selbst nur Pulsenergien im Nanojoule Bereich bereitstellt, können die Pulse mit einem regenerativen Verstärker bis zu einigen Millijoule Pulsenergie verstärkt werden. Während diese Laserquellen für Anwendungen mit hohen Abtragsraten pro Laserpuls geeignet sind, sind sie nicht optimal für die oben beschriebene Anwendung für Präzisionsschnitte.

Es ist bekannt, solche Laser für die refraktive Hornhautchirurgie zu verwenden. Übliche Pulsenergien betragen 5µJ bis 10µJ. Dadurch werden Kavitationsblasen erzeugt, deren Durchmesser 10µm bis 30µm beträgt. Durch diese Blasengröße wird eine Mikrorauhigkeit des erzeugten Schnittes in gleicher Größenordnung bewirkt. Bekannt ist andererseits, dass eine Mikrorauhigkeit in dieser Größenordnung nur unbefriedigende refraktive Ergebnisse gestattet.

In der US 5 993 438 wird ein Verfahren zum Bearbeiten von Augengewebe beschrieben. Die Laserstrahlpulse haben eine Pulslänge zwischen 100 fs und 10 ns und eine Pulsfrequenz von 0,1 kHz bis 0,1 MHz, wobei die Energie 200 GW/cm² bei einer Pulslänge von 50 ps und einem Fokusdurchmesser von 10 Mikrometern beträgt.

In der WO 01/54853 wird ein Verfahren und eine Vorrichtung zum Bearbeiten von Material beschrieben. Laserstrahlpulse, die ähnliche Merkmale wie die des Anspruchs 1 aufweisen, sind nur in Zusammenhang mit einer Oberflächenbearbeitung beschrieben.

In K. König et al., Optics Letters Vol. 26, No. 11 (2001*)* wurde beschrieben, wie auch mit Nanojoule-Pulsen aus einem Femtosekunden-Oszillator Schnitte in Gewebe ausgeführt werden können. Da dabei aber ein einzelner Laserpuls nicht zur Ausbildung einer Kavitationsblase führt, sondern mehrere, an die gleiche Stelle platzierte Pulse nötig sind, um eine Schnittwirkung zu erzielen, eignet sich dieses Verfahren nur für sehr feine Schnittfiguren im Mikrometermaßstab. Für den industriellen bzw. medizinischen Einsatz ist diese Laserquelle nicht geeignet.

Aufgabe der vorliegenden Erfindung ist es daher, eine Vorrichtung zur präzisen Bearbeitung von Material bereitzustellen, mit der diese Nachteile des Standes der Technik überwunden werden.

Die Erfindung ist in Anspruch1 definiert. Weitere vorteilhafte Ausgestaltungen sind in den abhängigen Ansprüchen angegeben.

Insbesondere wird die Aufgabe gelöst durch eine Vorrichtung zur präzisen Bearbeitung von Material, wobei diese Vorrichtung im zu bearbeitenden Material Kavitationsblasen erzeugt, deren Durchmesser weniger als 10µm beträgt. Um dies zu erreichen, wird ein gepulster Laserstrahl mit einer Pulsenergie von weniger als 5µJ auf einen Fokusdurchmesser von wenigen µm fokussiert. Vorzugsweise beträgt der Fokusdurchmesser etwa 3µm und die Pulsenergie 1µJ. Eine Verwendung einer Pulswiederholrate von mehr als 50 kHz gestattet eine sehr schnelle Bearbeitung. Dies ist insbesondere für die refraktive Hornhautchirurgie von großem Vorteil, weil damit eine Operationszeit von wenigen Sekunden bis ca. 1 Minute erreicht wird.

Eine Weiterbildung sieht vor eine Vorrichtung zur präzisen Bearbeitung von Material, umfassend ein gepulstes Lasersystem mit den erfindungsgemäßen Parametern als Strahlquelle, bei dem durch eine Strahleinrichtungen mit mindestens einem Mittel zur Strahlablenkung ein Arbeitsstrahl der Strahlquelle auf das Material applizierbar ist, wobei die Pulsaussendung mit der Strahlablenkung korreliert und wobei das Mittel zur Strahlablenkung Mittel zur Freigabe von Laserpulsen umfasst. Unter Freigabe wird dabei verstanden, dass der Laser für einen Laserimpuls freigegeben wird und der Laserimpuls ausgelöst wird, sobald der Laser entsprechend seiner maximalen Repetitionsrate erneut einen Laserimpuls abgeben kann. Unter Korrelation der Pulsaussendung mit der Strahlablenkung wird insbesondere verstanden, dass die Pulsaussendung erfolgen kann, wenn der Strahl auf einen bestimmten Punkt gelenkt wurde, die Pulsaussendung also in Abhängigkeit der Strahlablenkung angesteuert wird.

Die Vorrichtung zur präzisen Bearbeitung von Material umfasst ein gepulstes Lasersystem als Strahlquelle, wobei die Energie der Strahlung 100 nJ bis 5 µJ, beträgt. Der Fokusdurchmesser der Strahlung beträgt dabei vorzugsweise etwa 500 nm bis 10 µm, besonders bevorzugt 3µm bis 5µm. Die Pulsdauer der Strahlung beträgt vorzugsweise etwa 100 fs bis 1 ps, besonders bevorzugt 200 fs bis 500 fs.

Die Mittel zur Strahlformung und/oder Strahlablenkung bzw. allgemeiner formuliert die Strahlformungs- und -ablenkungssysteme können diffraktive oder refraktive Mikrooptiken oder adaptive Optiken oder klassische optische Systeme umfassen. Mit diffraktiven oder refraktiven Elementen kann man dabei mehrere klassische bzw. konventionelle optische Elemente ersetzen.

Die genannte Vorrichtung zur präzisen Bearbeitung von Material wird eingesetzt für die ophthalmologische Augenbehandlung, insbesondere zur Korrektur der Fehlsichtigkeit eines Auges. Die Vorrichtung kann zum Schneiden eines Flaps oder Lentikels in der Cornea zur Korrektur der Fehlsichtigkeit verwendet werden. Neben einem Schneiden des Lentikels können mit der erfindungsgemäßen Vorrichtung refraktive Strukturen in der Cornea, beispielsweise in Form flächenmäßig nebeneinander gesetzter Spots oder einer Punktwolke, erzeugt werden.

Ebenso können unmittelbar Laserschüsse zur Erzeugung refraktiver Strukturen gesetzt werden.

Geeignete Laserstrahlquellen sind Oszillator-Verstärker-Anordnungen, wobei für den Verstärker insbesondere Chirped-Pulse-Verstärker (CPA) oder Multipass-Verstärker geeignet sind.

Hinsichtlich der Bauform des modengekoppelten Oszillators sind insbesondere Scheibenlaseroszillatoren, Faserlaseroszillatoren, aber auch Stablaseroszillatoren geeignet.

Als Pumpquelle für die Lasermedien sind Halbleiter-Laserdioden aufgrund ihrer langen Lebensdauer, Zuverlässigkeit, Regelbarkeit und ihrer vergleichsweise geringen Herstellungskosten besonders vorzuziehen.

Bevorzugte Lasermedien in obigen Laserstrahlquellen sind dotierte Festkörpermaterialien, insbesondere Kristalle und Gläser. Beispielsweise sind dies YAG, Wolframate, Saphir und Fluoridgläser.

Diese Wirtsmaterialien können bevorzugt mit Neodym, Erbium, Titan, Chrom, Lithium oder Ytterbium dotiert werden. Alle diese Materialien zeichnen sich durch eine spektral breitbandige Laseremission im Spektralbereich von 600 nm bis 2000 nm aus und umfassen damit den für die refraktive Hornhautchirurgie besonders geeigneten Spektralbereich zwischen 800 nm und 1200 nm.

Die große spektrale Bandbreite der Laseremission der oben genannten Materialien unterstützt eine ultrakurze Laserpulsdauer zwischen 50 fs und 1 ps. Dabei ist es nicht erforderlich, dass der Laser selbst Pulse dieser Pulsdauer emittiert, dass aber die bevorzugte Impulsdauer von etwa 300 fs im zu bearbeitenden Werkstück bzw. auf seiner Oberfläche erreicht wird. Zu diesem Zweck umfasst die Vorrichtung ein optisches Modul welches dazu dient, die spektrale Phasenfunktion der Laserpulse geeignet zu beeinflussen. Dieses optische Modul erzeugt einen linearen Pre-Chirp, dessen Betrag dem linearen Chirp des optischen Systems angepasst ist. Dieses optische Modul kann bereits in einer Laserstrahlquelle geeignet integriert sein, insbesondere kann es mit dem Pulskompressor einer CPA-Laserstrahlquelle kombiniert oder mit diesem identisch sein.

Das gepulste Lasersystem ist eine Anordnung einer Laserstrahlquelle zur Erzeugung von fs-Pulsen und entsprechenden optischen Vorrichtungen, insbesondere Spiegel, Linsen, etc.

In einer Ausgestaltung der erfindungsgemäßen Vorrichtung ist vorgesehen, dass das Mittel zur Strahlablenkung im Scan-Modus betrieben werden. Der Arbeitsstrahl der Strahlquelle kann dabei auf in einer Dimension periodisch wiederkehrenden Bahnen abgelenkt werden, sodass beispielsweise kreisförmige Bahnen unterschiedlicher Durchmesser oder spiralförmige Bahnen erzeugt werden können. Die Bahnen des Arbeitsstrahles können durch eine rotierende oder in anderer Weise auf einer Bahn gehaltenen Vorrichtung, beispielsweise durch einen Spiegel, eine Linse, ein Gitter oder dergleichen, erzeugt werden. Die Mittel zur Strahlablenkung können Scanner, z.B. mechanische Scanner, umfassen, die auf vorgegebenen Bahnen bewegbar gelagert sind. Die vorliegende Erfindung nutzt schnelle Ablenksysteme, die den Laser auf den natürlichen Bahnen des Ablenksystems ablenkt, also z.B. auf Kreisbahnen oder Spiralbahnen bei rotierenden Ablenksystemen. Anstatt einzelne Positionen anzufahren und dort einen Laserimpuls auszulösen, sobald die vorgegebene Position erreicht ist und das Ablenksystem wieder ruht, wird die Bahn des Ablenksystems ohne Stops durchlaufen und die Pulse werden durch eine vorgewählte, über die Bahngeschwindigkeit der Fokusbewegung vorgegebene Repetitionsrate beginnend zu einem definierten Zeitpunkt abgegeben.

Sobald also die Fokusposition einen bestimmten Punkt erreicht hat, wird der Laser freigegeben und damit Laserpulse auf das Bearbeitungsgebiet gesendet. Dies führt zu einer Spur von Wirkvolumina, mithin durch den Laserfokus während der kurzen Pulsdauer modifizierte Stellen im Material, entlang einer im wesentlichen vordefinierten Bahn, die insbesondere dadurch ausgezeichnet ist, dass benachbarte Wirkvolumina in gleichbleibendem, vordefiniertem Abstand, beispielsweise in der Größenordnung des Durchmessers der Kavitationsblasen, platziert werden. Durch leichte Modifikation der natürlichen Bahn des Ablenksystems, z.B. durch eine leichte Reduktion des Kreisbahnradius, beispielsweise um den Betrag des Abstandes benachbarter Wirkvolumina, können weitere Spuren geschrieben werden, die sich zu einer Schnittfläche ergänzen. Beispielsweise können hier konzentrische Bahnen oder spiralförmige Bahnen oder dergleichen erzeugt werden. Bei Verwendung eines Ablenkspiegels kann dies beispielsweise durch eine Veränderung der Neigung bei gleichbleibender Rotation des Spiegels geschehen. Ziel ist es, die gewünschte Schnittfläche mit einem gleichmäßigen Raster von Wirkvolumina bzw. Laserfoki zu überdecken. Die natürlichen Bahnen des Ablenksystems können aufgrund der hohen Repetitionsrate des Lasersystems sehr schnell mit definiertem zeitlichem Ablauf durchfahren werden. Die Anpassung der zeitlichen Abfolge der Laserpulse führt dann zur gewünschten Überdeckung der Schnittfläche mit Laserschüssen.

Bei einem weiteren Ausführungsbeispiel der vorliegenden Erfindung sind weiter Strahleinrichtungen zur Strahlformung und/oder Strahlführung und/oder Strahlablenkung und/oder Strahlfokussierung vorgesehen. Durch diese Strahleinrichtungen kann der Strahl genau so auf das zu bearbeitende Material gelenkt und geleitet werden, wie es die geplante Anwendung erfordert. Die hier auf einen Fokusdurchmesser in der Größenordnung von 3 µm fokussierten ultrakurzen Laserpulse können insbesondere aufgrund ihrer geringen Pulsenergie von etwa 1µJ in einer kleinen, präzisen Kavitationsblase den Materialzusammenhalt lösen und/oder strukturelle Veränderungen im Material hervorrufen ohne benachbarte Gebiete im Material thermisch, akustisch oder mechanisch zu belasten. Für makroskopische Schnitte und Strukturen im Zentimetermaßstab wird der Laserfokus dreidimensional durch das zu bearbeitende Material gescannt. Der Anwendungsfall bestimmt, wie Strahlquelle, Strahlführung und -formung, Scanner, Scanalgorithmus und Fokussieroptik aufeinander abgestimmt werden, um eine hohe Bearbeitungsgeschwindigkeit bei gleichzeitig hoher Präzision zu erreichen.

Die Strahlformung geschieht dabei bevorzugt mittels eines Teleskops (bevorzugt Galilei-Teleskop mit Sammel- und Streulinse), das den Strahldurchmesser so aufweitet, dass der Laser auf einen entsprechend kleinen Fokus fokussiert werden kann. Bevorzugt wird ein Linsensystem verwendet, das die Abbildungsfehler des Teleskops weitgehend minimiert.

Die Strahlführung erfolgt bevorzugt durch Spiegel oder Spiegelpaare, mit denen der Strahl in die einzelnen Subkomponenten justiert werden kann.

Die Strahlablenkung können konventionelle Scanner bzw. mechanische Laserstrahl-Ablenksysteme wie Galvanometerspiegel im Close-Loop-Betrieb, etc. sein. Bevorzugt jedoch sind mechanische Scanner, die vorgegebene Bahnen (z.B. Kreisbahnen) abfahren und durch Triggerung der Strahlquelle an den vorgesehenen Positionen dadurch Laserpulse ausgelöst werden. So kann auf einem großen Bereich der Schnittfläche mit voller Repetitionsrate bei relativ langsamen Scannerbewegungen gearbeitet werden.

Die Strahlfokussierungseinrichtung dient dazu, im Fokus des Strahls auf oder innerhalb des Materials den Zusammenhalt des Materials aufzuheben (Photodisruption). Im Allgemeinen geht das mit einer lokalen Verdampfung des Materials einher. Bevorzugt wird der Laser hierfür auf einen Durchmesser im Mikrometerbereich fokussiert. Dies liegt nahe am Beugungslimit von Licht im sichtbaren bzw. nahen Infrarotbereich. Die Fokussieroptik weist daher bevorzugt eine hohe numerische Apertur und damit eine kurze Brennweite und eine große optische Öffnung (aufgeweiteter Laserstrahl Durchmesser) aus. Bevorzugt wird der von der Laserquelle ausgehende Strahl vor der Fokussierung auf das Material bzw. Gewebe im Durchmesser aufgeweitet. Die Systeme zur Strahlführung, -ablenkung und -fokussierung sind daher bevorzugt für einen großen Stahldurchmesser ausgelegt.

Laserquelle, Stahlablenkung (Scanner) und Fokussieroptik sind so aufeinander abgestimmt, dass präzise und schnelle Schnittführung im Wege der Fotodisruption ermöglicht wird. Dabei werden Laserspots mit einem Fokusdurchmesser von einigen 100 nm bis einigen µm mit einem Spotabstand in der Größenordnung des Kavitationsblasendurchmessers im Material platziert.

In einer besonders bevorzugten Ausführungsform sind die Strahleinrichtungen, insbesondere die Ablenkeinrichtungen, programmierbar. Durch die Abstimmbarkeit der einzelnen Strahleinrichtungen aufeinander und die Steuerung durch entsprechende Programme kann das System der Strahleinrichtungen zusammen mit dem gepulsten Lasersystem genau auf das Material und die Schnittanforderung eingestellt werden, für die es eingesetzt werden soll. So kann in Abhängigkeit der Transparenz und Brechkraft des zu bearbeitenden Materials sowie der Anforderung an Schnittgeometrie und Operationsdauer das Set an Parametern durch das Programm vorgewählt und abgestimmt werden.

In der vorliegenden Erfindung sind weiter Haltevorrichtungen zur Positionierung und/oder Fixierung des zu bearbeitenden Materials vorgesehen. Durch diese Haltevorrichtungen wird sichergestellt, dass die mikrometergenauen Strukturen, die durch den erfindungsgemäßen Laser hergestellt werden können, nicht durch unkontrollierbare Bewegungen des menschlichen Auges beeinträchtigt werden.

Fixiereinrichtungen für die medizinische Anwendungen am Auge müssen außerdem den jeweiligen biologischen Gegebenheiten angepasst sein. Die Fixierung des menschlichen Auges kann zum Beispiel mit Hilfe eines speziellen Adapters und eines Vakuum-Saugringes erfolgen.

Mit den beschriebenen hohen Repetitionsraten kann in Abstimmung mit den beschriebenen Pulsenergien geringen Betrages und der Ablenkeinrichtungen die Laserwirkung für die Photodisruption präzise lokalisiert werden. Hierdurch wird in einem scharf begrenzten Fokusvolumen das Materialgefüge zerstört, in dicht benachbarten Bereichen (von weniger als ein Mikrometer entfernt) findet im Allgemeinen keine Veränderung des Materials statt. Daraus ergibt sich eine hohe Bearbeitungspräzision (Mikrometergenauigkeit) bei Schonung benachbarter Materialregionen. Thermische und mechanische Beanspruchung der nicht bearbeiteten Regionen sind deutlich geringer als bei anderen Bearbeitungsmethoden.

Bei einer weiteren bevorzugten Ausführungsform der erfindungsgemäßen Vorrichtung ist durch die Strahleneinrichtung, insbesondere die Ablenkeinrichtung, ein Arbeitsstrahl der Strahlquelle in geometrisch vorbestimmbarer Form in zeitlich vorbestimmbarem Verlauf auf das Material applizierbar. Durch das Zusammenspiel der einzelnen Komponenten ist so möglich, Schnitte und Strukturierungen zu erzeugen. Zur Erzeugung eines Spots, in dem das Materialgefüge aufgelöst wurde, genügt im Allgemeinen ein Laserpuls mit definiertem Pulsparametem (Pulsenergie, Pulsdauer, Fokus). Für Schnitte und Strukturierung ist eine Vielzahl solcher Spots dicht nebeneinander zu platzieren. Der Abstand benachbarter Spots sollte am Ende der Prozedur in der Größenordnung der Kavitationsblasen liegen. Dafür kann der Laserfokus scannend über bzw. durch das Material bewegt werden. Der Laserfokus folgt in idealer Weise 3-dimensional mit Mikrometergenauigkeit einer vorgegebenen geometrischen Bahn. So ist es beispielsweise möglich, einen Schnitt in dem zu bearbeitenden Material dadurch zu erzeugen, dass eine beliebige Fläche, zum Beispiel eine Rechteckfläche benachbarter Mikrometerspots in dem Gewebe nacheinander scannend angesteuert wird. Dadurch wird genau in dieser Ebene der Materialzusammenhalt aufgelöst und dadurch ein "Schnitt" im Gewebe erzeugt. Genauso ist es möglich, den Laserfokus durch Kreisbewegungen des Scanners in einer Kreisbahn auf das zu bearbeitende Material zu applizieren. Durch eine sich anschließende helixförmige Führung des Bearbeitungsstrahls kann so beispielsweise eine Zylinderfläche aus dem Material herausgeschnitten werden. Da die Photodisruption bevorzugt in einem sehr engen Bereich stattfindet, kann der Laserstrahl auch im Gewebe wirken, ohne dass das vom Laserstrahl außerhalb des Fokus durchdrungene Material beschädigt wird. Auf diese Weise sind beliebige geometrische Bahnen und damit Formen durch Photodisruption in dem Material herausschneidbar.

Bei der refraktiven Hornhautchirurgie kann mit der erfindungsgemäßen Vorrichtung eine spezielle Schnittführung realisiert werden. Dabei wird kein traditioneller Flap präpariert, sondern das zuvor mit der erfindungsgemäßen Vorrichtung in der Cornea präparierte Lentikel über einen oder mehrere begrenzte seitliche Schnitte, welche ebenfalls mit der erfindungsgemäßen Vorrichtung erzeugt werden, am Umfang extrahiert. Zu diesem Zweck kann es vorteilhaft sein, dass Lentikel zuvor durch einen oder mehrere Schnitte mit der erfindungsgemäßen Vorrichtung zu zerteilen. Insbesondere ist eine Zerteilung derart sinnvoll, dass anschließend eine Entfernung der Teile mittels Absaugung mit einer Saug-Spül-Kanüle erfolgen kann.

Beim bevorzugten Ausführungsbeispiel der vorliegenden Erfindung ist eine Vorrichtung vorgesehen, bei der der gepulste Arbeitsstrahl durch die Strahlablenkungseinrichtung auf das Material applizierbar ist und währenddessen die Repetitionsrate der Pulse des Arbeitsstrahles modifizierbar ist. Durch das Vorsehen einer Einrichtung zur Modifizierung der Repetitionsrate bei gleichzeitiger Strahlführung des Arbeitsstrahles über das zu bearbeitende Material kann auf diese Weise elegant ein gleichmäßiges Spotmuster auf dem zu behandelnden Material erzeugt werden, auch wenn der Strahl unter verschiedenen Winkeln bzw. verschieden schnell durch die Ablenkeinrichtung auf das zu bearbeitende Material gerichtet wird. Ein besonders augenfälliger Vorteil wird beispielsweise dann erreicht, wenn die Ablenkeinrichtung den Strahl in Kreisbahnen auf das zu bearbeitende Material lenkt und diese Kreisbahnen mit einer speziellen Umlauffrequenz der Ablenkeinrichtung, insbesondere beispielsweise der Ablenkspiegel, erzeugt wird. Wird bei einer Umlauffrequenz von beispielsweise 50Hz der Laserstrahl auf einer Kreisbahn von 1cm Durchmesser bei einer Repetitionsrate von 300kHz geführt, dann werden auf jeder Kreisbahn pro Umlauf gleichmäßig verteilt 60000 Spots gesetzt. Wenn der Strahl dann auf einem Kreis von nur 0,5cm Durchmesser mit derselben Frequenz der Ablenkeinrichtung geführt wird, kann durch Erniedrigung der Repetitionsrate des gepulsten Arbeitsstrahles der gleiche Abstand der einzelnen Spots voneinander auf dem zu bearbeitenden Material erzeugt werden, wie bei der Strahlführung auf der größeren Kreisbahn. Durch eine entsprechende Modifikation der Repetitionsrate in Abhängigkeit der durch die Ablenkeinrichtung abgefahrenen Geometrie lassen sich so beliebige geometrische Muster mit einem im Wesentlichen gleich bleibenden Spotabstand auf dem zu bearbeitenden Material erzeugen. Beispielsweise können Spiralen abgefahren werden, bei denen von außen nach innen bei gleichbleibender Umlauffrequenz der Ablenkeinrichtung die Repetitionsrate immer weiter abnimmt. Daneben sind auch beliebige andere geometrische Formen denkbar. Ist eine konstante Beabstandung der einzelnen Spots auf dem Material gerade nicht beabsichtigt, sondern soll vielmehr in einem speziellen Bereich eine höhere Spotdichte und in einem weiteren Bereich eine niedrigere Spotdichte erreicht werden, so kann dies ebenfalls durch Kombination der gewählten Parameter für die Repetitionsrate des Arbeitsstrahles und die Frequenz bzw. den örtlichen Verlauf der Ablenkeinrichtung erzeugt werden. So ist es bevorzugt auch möglich, graduell unterschiedliche Bereiche mit verschiedener Fokusdichte zu erzeugen. Beispielsweise kann bei einem Kreis das Zentrum einen sehr niedrigen Fokusabstand aufweisen während der Fokusabstand zum Rand hin immer größer wird.

Bevorzugt wird der gepulste Laserstrahl mittels einer Ablenkeinrichtung auf das zu bearbeitende Material gelenkt und in Abhängigkeit des hierdurch auf dem Material erzeugten Spotmusters die Repetitionsrate der Pulse des Laserstrahls modifiziert. Auf diese Weise kann jedes beliebige Spotmuster und insbesondere jede beliebige Beabstandung der einzelnen Spots voneinander in der gewünschten Geometrie auf dem zu bearbeitenden Material erzeugt werden. Besonders bevorzugt werden die Spotmuster so auf dem zu bearbeitenden Material verteilt, dass die Kavitationsblase jedes einzelnen Spots, die durch Photodisruption entsteht, genau benachbart zu der Kavitationsblase des nächsten Spots gesetzt wird. Auf diese Weise entsteht dann ein gewünschtes Schnittmuster direkt benachbarter Kavitationsblasen. Für spezielle Anwendungsfälle kann es auch gewünscht sein, die Spots noch enger zu setzen. Dies ist beispielsweise dann empfehlenswert, wenn das zu bearbeitende Material sich nach einer gewissen Zeit wieder erneuert und die Ablösung des Materials für eine spezielle Zeit sichergestellt werden soll, bevor beispielsweise der Bohrkern oder ein sonst herausgeschnittenes Stück des zu bearbeitenden Materials entfernt werden kann. Ebenso ist es denkbar, dass die Spots zuerst mit einer größeren Beabstandung gesetzt werden, um in einem folgenden Schritt die Lücken zwischen den Spots zu füllen und dadurch ein gewünschtes Muster von Kavitationsblasen zu bilden.

Die erfindungsgemäße Vorrichtung kann verwendet werden zur refraktiven Chirurgie durch Bearbeitung der Cornea des Auges.

Im Folgenden sollen weitere vorteilhafte Ausgestaltungen der Erfindung an Hand von Zeichnungen erläutert werden.
Fig. 1 zeigt eine schematische Darstellung eines Lasers,
Fig. 2 zeigt ein Ausführungsbeispiel eines erfindungsgemäßen Lasers mit Operationsmikroskop und zu bearbeitendem Auge;
Fig. 3 zeigt eine schematische Darstellung von einigen Beispielen möglicher Schnittmustern, die mit einem Lasersystem ausgeführt werden können;
Fig. 4 zeigt schematisch eine Detailansicht einer Folge von Laserspots auf Kreislinien und
Fig. 5 zeigt den zeitlichen Verlauf von Folgen von Laserpulsen im und außerhalb des Laserresonators.
Fig. 6 zeigt die Schnittführung zur Erzeugung eines Lentikels im Schnitt durch die Cornea
Fig. 7 zeigt den Vorgang der Extrahierung des geschnittenen Lentikels durch einen kleinen seitlichen Schnitt,
Fig. 8 zeigt das geschnittene Lentikel in der Draufsicht der Cornea,
Fig. 9 zeigt eine weitere Form der Schnittführung wobei das Lentikel zerteilt wird und durch zwei seitliche Schnitte extrahiert werden kann und
Fig. 10 zeigt eine weitere Ausbildung des Verfahrens wobei die Linse in viele Teile zerteilt wird, welche mit einer Saug-Spül-Einrichtung entfernt werden.

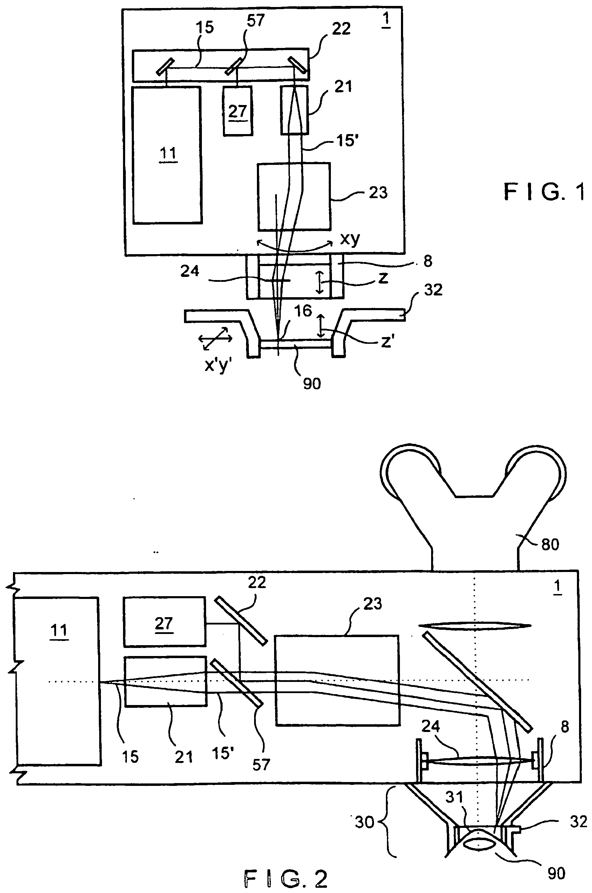

In Figur 1 ist eine schematische Darstellung der einzelnen Komponenten eines Lasersystems, das nicht die Merkmalskombination des Anspruchs 1 realisiert, dargestellt. Die Bearbeitungsvorrichtung 1 umfasst als Strahlquelle 11 eine fs-Laserstrahlquelle. Der Laserstrahl 15 wird über Spiegel und einen Strahlteiler 57 auf eine Strahlaufweitungsoptik 21 ausgekoppelt. Der aufgeweitete Laserstrahl 15' wird dann über eine Strahlablenkungseinrichtung wie beispielsweise einen Scanner in XY-Richtung auf eine Strahlfokussierungseinrichtung 24 gelenkt. Diese ist in der Z-Achse verschiebbar und erlaubt so die Verschiebung des Fokuspunktes durch Verschiebung der Strahlfokussierungseinrichtung entlang des Pfeiles Z. Alternativ kann ein fokussierendes optisches System mit veränderlicher Brennweite verwendet werden, um die Fokusposition in Z-Richtung kontrolliert zu verschieben. Der fokussierte Laserspot 16 wird so auf das zu bearbeitende Material 90 gelenkt, das durch eine Fixierungsvorrichtung 32 in seiner Position gehalten wird. Das Material 90 ist hier eine zu bearbeitende Kontaktlinse. Der Spot 16 kann auch durch Verschieben der Fixierungsvorrichtung 32 in Richtung XY' bzw. Z' auf bzw. in dem Material ausgerichtet werden.

Durch die Bearbeitungsvorrichtung 1 wird der von der Strahlquelle 11 erzeugte Laserstrahl 15 auf das Material 90 fokussiert. Ein Fokusdurchmesser von wenigen Mikrometern kann dadurch erreicht werden, dass der Laserstrahl 15 mit einem Strahldurchmesser von einigen Millimetern durch eine Optik mit einigen Zentimetern Brennweite fokussiert wird. Beispielsweise ergibt sich für ein gaußförmiges Strahlprofil ein Fokusdurchmesser von drei Mikrometern, wenn ein Laserstrahl der Wellenlänge 1000 nm und einem Strahldurchmesser von 10 mm mit einer Brennweite von 50 mm fokussiert wird.

Im Allgemeinen besitzt der Laserstrahl 15 am Ausgang der Strahlquelle 11 einen geringeren Strahldurchmesser als zur optimalen Fokussierung notwendig ist. Mit einer Strahlaufweitungsoptik 21 kann der Strahldurchmesser den Erfordernissen angepasst werden. Bevorzugt kann als Strahlaufweitungsoptik 21 ein auf unendlich eingestelltes Teleskop nach Galilei (Zerstreuungslinse plus Sammellinse) eingesetzt werden. Hierbei entsteht kein Zwischenfokus, der unter Umständen schon zu einem optischen Durchbruch in Luft führen könnte. Damit ist die verbleibende Laserenergie höher und das Strahlprofil gleichbleibend gut. Bevorzugt ist die Verwendung von Linsensystemen, die zu optimalen Abbildungseigenschaften des Teleskops führen. Durch Justage des Teleskops können auch Fertigungsschwankungen in der Strahldivergenz der Strahlquelle 11 ausgeglichen werden.

In diesem Beispiel wird der Laserfokus scannend über bzw. durch das Material bewegt. Der Laserfokus bzw. Laserspot 16 wird so dreidimensional mit Mikrometergenauigkeit gescannt. Der aufgeweitete Laserstrahl 15' wird senkrecht zur ursprünglichen Strahlrichtung durch eine Ablenkeinrichtung 23 abgelenkt. Hierbei verschiebt sich die Lage des Fokus 16 nach der Fokussieroptik 24 senkrecht zur ursprünglichen Strahlrichtung. Damit kann der Fokus in einer Fläche, die im Wesentlichen eben und senkrecht zur Laserstrahlrichtung ist (X/Y-Richtung) bewegt werden. Die Bewegung parallel zur Strahlrichtung (Z-Richtung) kann zum einen durch bewegen des Werkstücks erfolgen (siehe Pfeil Z'). Die Scan-Algorithmen sind dann bevorzugt so ausgelegt, dass das Werkstück nur langsam bewegt werden muss und die schnellen Scannbewegungen von der Ablenkeinheit ausgeführt werden. Zum anderen kann auch die Fokussieroptik parallel zur Laserstrahlrichtung bewegt werden (Pfeil Z), um damit den Fokus in Z-Richtung zu senken. Insbesondere bei medizinischen Applikationen ist die zweite Methode bevorzugt, da der Patient im Allgemeinen nicht schnell genug bewegt werden kann.

Das bearbeitete Material 90 wird relativ zum Lasergerät in einer Fixier- und Justagevorrichtung 32 fixiert. Bevorzugt wird hier die Fixiervorrichtung senkrecht und parallel zur Strahlrichtung justiert, um das Schnittmuster an die vorgesehene Stelle im Material 90 platzieren zu können. Ein mit dem bearbeitenden Laserstrahl 15, 15' kolinearer sichtbarer Laserstrahl aus einem Pilotlaser 27 unterstützt hierbei die Justierung.

Zur Strahlführung und zur Feinjustage der Strahllage zwischen den einzelnen Komponenten sind Spiegel bzw. Spiegelpaare 22 vorgesehen. Die Beschaffenheit der Spiegel wird bevorzugt so gewählt, dass der bearbeitende Laserstrahl diesen nicht zerstört, die Spiegel hoch reflektierend für die Wellenlänge des Bearbeitungslasers und hinreichend reflektierend für den Pilotlaser sind. Bevorzugt wird die Beschichtung so gewählt, dass der Spiegel die Laserpulsdauer nicht wesentlich verlängert. Besonders bevorzugt wird mindestens einer der Spiegel ein sogenannter "Chirped Mirror" sein, mit dem die Dispersion aller im Strahlengang vorhandenen Optiken kompensiert werden kann, um optimal kurze Pulse im Bearbeitungsfokus zu erzielen.

In Figur 2 ist ein Ausführungsbeispiel der erfindungsgemäßen Laserbearbeitungsvorrichtung mit Operationsmikroskop gezeigt. Der Aufbau entspricht im Wesentlichen dem Aufbau in Figur 1. Gleiche Teile sind mit gleichen Bezugszeichen gekennzeichnet. Als Material 90 ist hier die Hornhaut des menschlichen Auges vorgesehen. Es soll nun beispielhaft dieses Lasergerät detailliert beschrieben werden, mit dem präzise Schnitte in der Hornhaut des menschlichen Auges eingebracht werden können. Dabei soll eine kreisförmige Fläche, die der Krümmung der Hornhaut folgt und zur optischen Achse des Auges zentriert ist, mit fs-Laserpulsen innerhalb der Hornhaut geschnitten werden. Durch einen kreissegmentförmigen Randschnitt von der Kreisfläche bis zur Außenseite der Hornhaut entsteht ein Hornhautlappen (Flap), der nach dem Laserschnitt zur Seite geklappt werden kann.

Solch ein Flap dient zur Vorbereitung einer LASIK-Operation, bei der durch Laserabtrag die Dicke der Hornhaut so variiert wird, dass refraktive Fehler des Auges kompensiert werden. Bisher wird dieser Schnitt mit einem mechanischen Keratom durchgeführt, was ein hohes Maß an Übung beim Arzt voraussetzt und risikobehaftet ist. Zusätzlich kann durch eine weitere gekrümmte Kreisfläche, die zusammen mit der ersten Kreisfläche des Flaps ein Lentikel umschließt, das nach Aufklappen des Flaps entnommen werden kann, im gleichen Arbeitsgang eine refraktive Korrektur der Hornhaut erfolgen.

Bei der besonderen Ausgestaltung der Erfindung wird das Auge durch einen Saugring 32 an ein Kontaktglas 31 gedrückt, das entweder eben ist, oder bevorzugt der Krümmung der Hornhaut im Wesentlichen angepasst ist. Der Saugring ist fest mit dem Austrittsfenster des Lasergerätes verbunden, was für eine definierte Lage der Hornhaut relativ zum Laserfokus sorgt. Der aufgeweitete Femtosekunden-Laserstrahl wird mit einer Optik 24 in die Hornhaut fokussiert. Ein Strahlteiler, der für die Laserwellenlänge hochreflektierend und für sichtbares Licht transmittierend ist, spiegelt den Laserstrahl in den Strahlengang eines Operationsmikroskopes ein, das zur Beobachtung und Zentrierung des Auges dient. Die Fokussieroptik 24 bildet dabei einen Teil des Mikroskopobjektives. Zusammen mit einer bündelnden Optik kann ein reelles Zwischenbild der Hornhaut erzeugt werden, das man sich mit dem Stereo-Okular 80 räumlich anschauen kann. Die Strahlablenkeinheit 23 lenkt den aufgeweiteten Laserstrahl 15 senkrecht zu dessen Ausbreitungsrichtung aus. Somit kann der Laserfokus auf unterschiedliche Punkte in der Hornhaut gerichtet werden. Die Fokustiefe kann durch verschieben der Fokussieroptik 24 längs der optischen Achse oder durch Anpassung der Brennweite der Fokussieroptik variiert werden.

Vorzugsweise werden mit der Ablenkeinheit Kreisbahnen abgefahren. Zum Schneiden der Kreisfläche wird der Kreisradius von Kreisbahn zu Kreisbahn verringert und die Repetitionsrate so angepasst, dass ein einheitlicher Spot-Abstand beibehalten wird. Die Fokustiefe wird von Kreisbahn zu Kreisbahn so angepasst, dass der Schnitt der Krümmung der Hornhaut folgt. Sollen astigmatische Korrekturen der Sehkraft (Zylinderkorrektur) eingebracht werden, kann die Fokustiefe während der Kreisbahn zweimal auf und ab bewegt werden, so dass ein Lentikel mit Zylinderlinsenanteil entsteht. Für die Flapkante wird bei festem Radius die Fokustiefe vom Flapboden langsam bis zur Außenseite der Hornhaut verschoben, so dass ein Zylindermantel entsteht. Auf einem Bogenstück der dabei beschriebenen Kreise muss der Laserstrahl unterbrochen werden, um einen "Hinge", an dem der präparierte Flap festgehalten wird, zu belassen. Dazu wird einfach das Auskoppeln von Laserpulsen aus der Strahlquelle 11 unterbrochen.

Die Strahlquelle 11 ist eine Femtosekunden-Strahlquelle mit den oben beschriebenen Parametern, der bevorzugt direkt diodengepumpt und damit einfach und zuverlässig ist. Der emittierte Laserstrahl 15 wird bevorzugt mit einem Galilei-Teleskop auf 1-2 cm Strahldurchmesser aufgeweitet. Kollinear zum aufgeweiteten Laserstrahl 15 wird ein sichtbarer Laserstrahl aus einem Pilotlaser 27 überlagert, der dann zusammen mit dem Bearbeitungslaserstrahl gescannt und fokussiert wird. Der Strahlteiler 57 ist für diesen Zweck transparent für die Femtosekunden-Laserwellenlänge und reflektierend für den Pilotstrahl.

Die Vielfalt der möglichen Schnittfiguren hängt nur von den Scanalgorithmen ab. Prinzipiell ist ein Lasergerät wie beschrieben zu einer Vielzahl von Applikationen (beispielsweise für refraktive Korrekturen der Sehkraft), bei denen Schnitte oder Strukturumwandlungen innerhalb der transparenten Hornhaut vorgenommen werden sollen, geeignet. Damit übertrifft die Erfindung selbst in diesem kleinen Teilbereich der Anwendung in Universalität und Präzision (Schonung von umliegendem Gewebe) bestehende Technologien bei weitem.

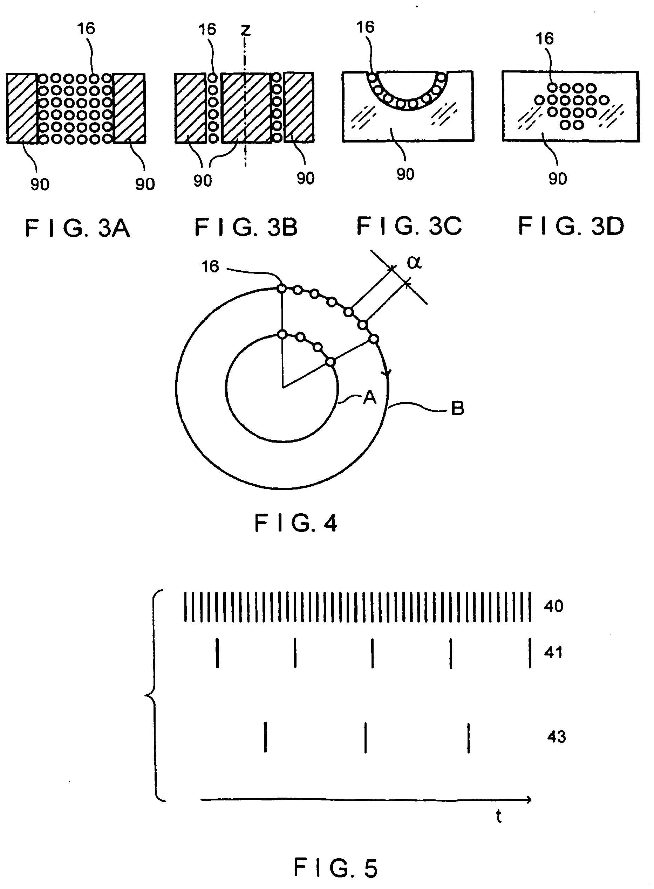

In Figur 3 sind in den Unterdarstellungen 3 a bis d Anwendungsbeispiele von Schnittgeometrien gezeigt, die mit einem Lasersystem realisiert werden können. Diese Anwendungen sind nur beispielhaft - es können beliebige weitere Geometrien realisiert werden. Im Fokus 16 des Lasers wird der Zusammenhalt des Materials 90 aufgehoben (Photodisruption). Im Allgemeinen geht das mit einer lokalen Verdampfung des Materials einher. Nach der Einwirkung des Laserpulses ist in einem kleinen Volumen, der Kavitationsblase (im Folgenden auch Spot 16 genannt) das Materialgefüge dauerhaft oder für einen bis mindestens zum Ende der Bearbeitungsdauer dauernden Zeitraum aufgehoben. Der Einsatz eines stark fokussierten Femtosekunden-Lasers bietet damit die präziseste Lokalisierung der Laserwirkung. In dem scharf begrenzten Fokusvolumen wird damit das Materialgefüge zerstört, während es in dicht benachbarten Bereichen (schon weniger als ein Mikrometer entfernt) im Allgemeinen keine Veränderung des Materials stattfindet. Daraus ergibt sich eine hohe Bearbeitungspräzision bei Schonung benachbarter Materialregionen.

Für Schnitte und Strukturierungen werden eine Vielzahl von einzelnen Spots, die das Materialgefüge auflösen, dicht nebeneinander platziert. Der Abstand benachbarter Spots sollte am Ende der Prozedur in der Größenordnung des Spotdurchmessers liegen. In Figur 3 a wird ein vorbestimmtes Volumen (z.B. eine Bohrung im Material) durch vollständiges Ausfüllen des abzutragenden Volumens mit einzelnen Spots 16 generiert. Bei einem solchen nicht transparenten Material geht man dabei schichtweise beginnend mit der dem Laser zugewandten Schicht von Spots vor.

In Figur 3b wird nur der Rand der Bohrung mit Spots überdeckt. Es soll hier ein Schnitt durch das Material gezeigt sein. Die Spots 16 sollen rotationssymmetrisch um die gestrichelt eingezeichnete Achse Z angeordnet sein. Auf diese Weise wird ein Bohrkern in der Mitte des bearbeiteten Materials 90 erzeugt. Der Bohrkern kann anschließend als zusammenhängendes Stück entnommen werden. Die benötigte Anzahl von Laserpulsen verringert sich damit insbesondere bei großen Querschnittsflächen der Bohrung erheblich im Vergleich zur Figur 3 a.

In Figur 3c ist eine Unterschneidung in einem transparenten Material 90 gezeigt. Da die Strahlung vom Material 90 nicht absorbiert wird, sind zusammenhängende Materialstücke durch Platzierung von Spots auf der Schnittkante aus dem Material herauslösbar, wenn dieses an die Oberfläche grenzt.

In Figur 3d ist gezeigt, wie in einem transparenten Material je nach Beschaffenheit des Materials Hohlräume bzw. Strukturierungen (z.B. Änderungen der optischen Eigenschaften) erzeugt werden können.

Für makroskopische Schnittfiguren (im Zentimeterbereich) werden einige Millionen Laserspots benötigt, selbst um nur die Schnittfläche (wie in Figuren 3b und c) dicht genug mit Spots zu überdecken. Für viele Anwendungen (insbesondere medizinische Applikationen) ist es vorteilhaft, die Bearbeitungs- bzw. Behandlungszeit so gering wie möglich zu halten. Die Strahlquelle des Lasergeräts ist daher erfindungsgemäß in der Lage, Laserpulse mit einer hohen Repetitionsrate abzugeben. In Figur 4 wird schematisch ein Ausschnitt aus einem möglichen Scanmuster gezeigt, bei dem die einzelnen von einzelnen Laserpulsen bearbeiteten Spots 16 entlang von Bahnen angeordnet sind, die vom Scanner kontinuierlich abgefahren werden können. Um bei hohen Repetitionsraten der Strahlquelle 11 einen hinreichenden großen Spotabstand zu erzielen, wird der Fokus in mindestens einer von drei Scandimensionen sehr schnell bewegt. Die Scan-Algorithmen werden daher bevorzugt so ausgelegt, dass die Spots entlang von Bahnen, die den natürlichen Bewegungen der Ablenkeinheit entsprechen, platziert werden. Die Bewegung in den anderen zwei Dimensionen kann dann relativ langsam erfolgen. Die natürlichen Bahnen der Ablenkeinheit können z.B. Kreisbahnen sein, die die Ablenkeinheiten mit festen Umlauffrequenzen abfahren kann. Das kann z.B. durch rotierende optische Elemente in der Ablenkeinheit erfolgen. Der Radius der Kreisbahn und die Fokustiefe (Z-Richtung) sind dann die langsam veränderbaren Scangrößen. Diese Variante eignet sich besonders, wenn rotationssymmetrische Schnittfiguren erzeugt werden müssen. Die Repetitionsrate des Lasers läßt sich dann besonders effektiv nutzen, wenn die Umlauffrequenz der Kreisbahnen so gewählt wird, dass bei den größten abzufahrenden Kreisbahnen (B) die volle Repetitionsrate der Strahlquelle zum gewünschten Spotabstand d führt. Werden die Kreisbahnen beim Abfahren des Schnittmusters kleiner im Radius (A), kann die Repetitionsrate der Quelle entsprechend verringert werden, so dass sich wieder der optimale Spotabstand ergibt. Diese Anpassung der Repetitionsrate ist bei der beschriebenen Laserstrahlquelle ohne weiteres möglich. Eine Anpassung der Umlauffrequenz an die Repetitionsrate der Quelle kann technologisch schwieriger sein, insbesondere wenn diese kontinuierlich für jede Kreisbahn (A, B) erfolgt. Für eine Verringerung der Bearbeitungszeit kann aber eine Anpassung der Umlauffrequenz in wenigen Schritten an die kleineren Kreisbahnen von Vorteil sein.

In Figur 5 sind mögliche Folgen von Laserpulsen in und außerhalb einer Oszillator-Verstärker-Anordnung dargestellt. Die Umlauffrequenz der Laserpulse im Oszillator 40 hängt nur von der Resonatorlänge ab und ist für eine bestimmte Strahlquelle vorgegeben und liegt bei Resonatorlängen von wenigen Metern um 100 MHz. Bei der hier dargestellten regenerativen Verstärkung werden beispielsweise die Pulse 41 in den Verstärker eingekoppelt und verstärkt. Wird eine geringere Repetitionsrate gewünscht, erfolgt die Verstärkung der Pulse 43. Eine Veränderung der Repetitionsrate der verstärkten Laserimpulse ist auf diese Weise aufwandgering zu realisieren.

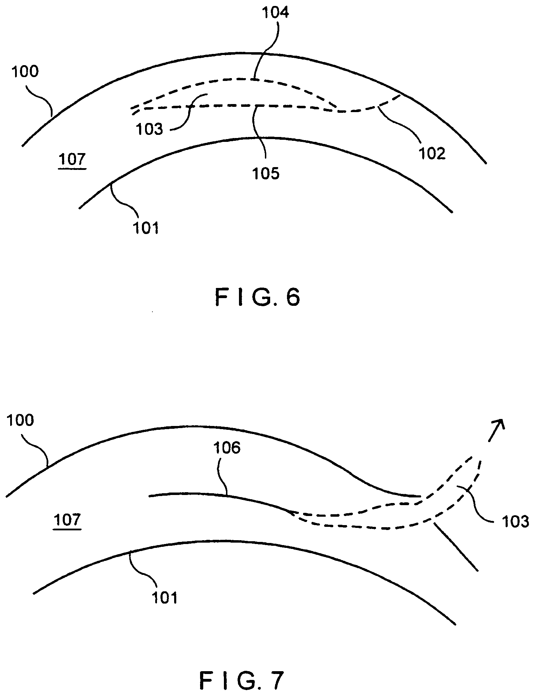

Figur 6 zeigt eine Schnittdarstellung der menschlichen Hornhaut 107 mit Vorderseite 100 und Rückseite 101. Das Lentikel 103 wird durch die beiden flächigen Schnitte 104 und 105 gebildet. Ein kleiner seitlicher Schnitt 102, welcher bis zur Hornhautvorderfläche 100 führt ermöglicht die Extraktion des Lentikels 103. Diese Extraktion ist in Figur 7 dargestellt. Der verbleibende Hohlraum kollabiert 106.

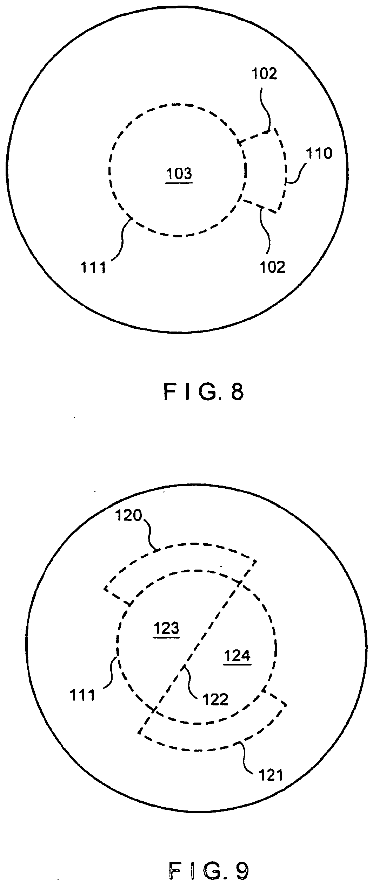

Figur 8 stellt die Hornhaut in der Draufsicht dar. Erkennbar ist die Berandung 111 des Lentikels 103, sowie die an die Hornhautvorderfläche führenden Schnitte 102. Entlang der Linie 110 wird die Hornhautvorderfläche durchtrennt und die Extraktion der Linse ermöglicht.

Figur 9 stellt eine weitere bevorzugte Form der Schnittführung dar. Dabei wurde das Lentikel in zwei Teile 123 und 124 durch einen Schnitt 122 zerteilt. Statt eines einzigen Extraktionsschnittes 110 werden hier zwei Extraktionsschnitte 120 und 121 angebracht. Im nachfolgenden wird das Linsenteil 123 durch den Extraktionsschnitt 120 und das Linsenteil 124 durch den Extraktionsschnitt 121 entfernt.

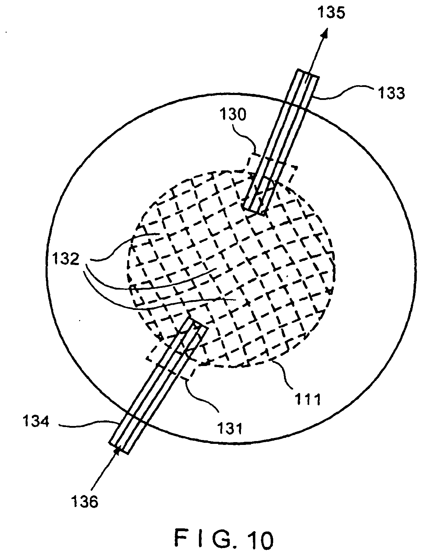

Figur 10 stellt eine weitere Ausprägung des Verfahrens dar. In dieser Ausprägung wird das durch den Rand 111 berandete Lentikel in viele kleine Fragmente 132 zerschnitten. Diese können nun mit Hilfe einer Kanüle 133, die vorzugsweise einen an die Fragmentgröße angepassten Durchmesser besitzt, abgesaugt werden. Dieser Vorgang kann durch eine Spüleinrichtung über eine zweite Kanüle 134, die in einen gegenüberliegenden Kanal oder auch den gleichen Kanal eingeführt wird, unterstützt werden. Das Spülmittel 136, 135 ist vorzugsweise isotonische Kochsalzlösung wobei auch andere Lösungen eingesetzt werden können. Dieses Verfahren realisiert eine minimalste Schwächung der Cornea durch diese Methode der refraktiven Laserchirurgie.

Die Erfindung wurde an Hand bevorzugter Ausführungsbeispiele erläutert. Fachmännische Weiterentwicklungen führen nicht zu einem Verlassen des durch die Ansprüche definierten Schutzumfangs.

## Patentansprüche

1. Vorrichtung zum präzisen Bearbeiten von Material, nämlich der menschlichen Augenhornhaut, mit
- einem gepulsten Laser, wobei der Laser Laserstrahlpulse mit einer Pulslänge zwischen 50 fs und 1 ps und einer Pulsfrequenz von 100 kHz bis 1 MHz, nicht aber 100 kHz bereitstellt, wobei die Energie der einzelnen Laserstrahlpulse 100 nJ bis 5 µJ beträgt,
- Haltevorrichtungen zur Positionierung und/oder Fixierung des zu bearbeitenden Materials und
- einer Strahlfokussierungseinrichtung, welche die Laserstrahlpulse in das Innere des Materials fokussiert,
- wobei der gepulste Laser und die Strahlfokussierungseinrichtung so ausgebildet sind, daß die Laserstrahlpulse im innerhalb des Materials gelegenen Fokus eine Photodisruption bewirken,
- wobei der gepulste Laser eine Oszillator-Verstärker-Anordnung ist, deren Verstärker ein Faserlaserverstärker ist, und
- wobei die Vorrichtung ein optisches Modul umfasst, das eine spektrale Phasenfunktion der Laserpulse beeinflusst und einen linearen Pre-Chirp erzeugt, dessen Betrag dem linearen Chirp des optischen Systems angepasst ist.

2. Vorrichtung nach Anspruch 1, wobei weiter Strahleinrichtungen zur Strahlformung und/oder Strahlführung und/oder Strahlablenkung vorgesehen sind.

3. Vorrichtung nach Anspruch 2, wobei die Strahleinrichtungen programmierbar sind.

4. Vorrichtung nach einem der Ansprüche 2 bis 3, wobei durch die Strahleinrichtungen die Laserstrahlpulse in geometrisch vorbestimmbaren Formen in zeitlich vorbestimmbarem Verlauf in das Material applizierbar sind.

5. Vorrichtung nach Anspruch 4, wobei die Laserstrahlpulse durch die Strahlablenkungseinrichtung auf das Material applizierbar sind und währenddessen die Pulsfrequenz modifizierbar ist.

## Claims

1. Apparatus for precise processing of material, namely of the cornea of the human eye, the apparatus comprising
- a pulsed laser, wherein the laser provides laser beam pulses having a pulse length between 50 fs and 1 ps and a pulse frequency from 100 kHz to 1 MHz, but not of 100 kHz, wherein the energy of the individual laser pulses is 100 nJ to 5 µJ,
- holding devices for positioning and/or fixing the material to be processed and
- a beam focusing device which focuses the laser pulses into the inside of the material,
- wherein the pulsed laser and the beam focusing device are configured such that the laser pulses cause a photodisruption in the focus lying inside the material,
- wherein the pulsed laser is an oscillator amplifying arrangement, which amplifier is a fiber laser amplifier,
- wherein the apparatus includes an optical module which influences a spectral phase function of the laser pulses and generates a linear pre-chirp, the amount of which is adapted to the linear chirp of the optical system.

2. Apparatus according to claim 1, wherein further beam devices for beam shaping and/or beam guiding and/or beam deflection are provided.

3. Apparatus according to claim 2, wherein the beam devices are programmable.

4. Apparatus according to any of claims 2 through 3, wherein by means of the beam devices the laser beam pulses are applicable in geometrically pre-determinable shapes and in timely pre-determinable progression into the material.

5. Apparatus according to claim 4, wherein the laser beam pulses are applicable by means of the beam deflection device while in the meantime the pulse frequency is modifiable.

## Revendications

1. Dispositif pour le traitement de précision de matière, à savoir de la cornée de l'œil humain, comprenant
- un laser pulsé, le laser fournissant des impulsions de faisceau laser ayant une longueur d'impulsion comprise entre 50 fs et 1 ps et une fréquence d'impulsion de 100 kHz à 1 MHz, mais pas de 100 kHz, l'énergie des impulsions de faisceau laser individuelles étant de 100 nJ à 5 µJ,
- des dispositifs de fixation pour le positionnement et/ou la fixation de la matière à traiter et
- un dispositif de focalisation de faisceau qui focalise les impulsions de faisceau laser à l'intérieur de la matière,
- le laser pulsé et le dispositif de focalisation de faisceau étant réalisés de telle sorte que les impulsions de faisceau laser provoquent une photo-disruption dans le foyer situé à l'intérieur de la matière,
- le laser pulsé étant un agencement d'oscillateur-amplificateur dont l'amplificateur est un amplificateur de laser à fibres optiques,
- le dispositif comprenant un module optique qui influence une fonction de phase spectrale des impulsions laser et qui génère une préchirp linéaire dont la valeur est adaptée à la chirp linéaire du système optique.

2. Dispositif selon la revendication 1, dans lequel sont prévus en outre des dispositifs pour rayonnement destinés à façonner et/ou à guider et/ou à dévier le rayonnement.

3. Dispositif selon la revendication 2, dans lequel les dispositifs de rayonnement sont programmables.

4. Dispositif selon l'une quelconque des revendications 2 à 3, dans lequel, par les dispositifs de rayonnement, les impulsions laser sont applicables dans le matériau dans des formes géométriquement prédéfinissables selon un déroulement prédéfinissable dans le temps.

5. Dispositif selon la revendication 4, dans lequel les impulsions de rayonnement laser sont applicables sur le matériau par le dispositif de déviation du rayonnement et dans lequel, pendant ce temps, la fréquence des impulsions est modifiable.
